# EUROPEAN PATENT APPLICATION

(11) **EP 4 516 400 A1**
(43) Date of publication of application: **05.03.2025**
(21) Application number: 23795517.4
(22) Date of filing: 27.04.2023
(51) Int. Cl.: B01J 38/00, B01J 38/12

(54) **CATALYTIC CRACKING CATALYST REGENERATION METHOD AND SYSTEM USING BIO-BASED LIQUID-PHASE FUEL**

(30) Priority: 29.04.2022 CN 202210474591; 29.04.2022 CN 202210475997; 29.04.2022 CN 202210476000; 29.04.2022 CN 202210474580; 16.09.2022 CN 202211132128
(71) Applicant: China Petroleum & Chemical Corporation, Beijing 100728 (CN); Sinopec Research Institute of Petroleum Processing Co., Ltd., Beijing 100083 (CN)
(72) Inventor: YANG, Wenjie, Beijing 100083 (CN); XU, Youhao, Beijing 100083 (CN); WANG, Xin, Beijing 100083 (CN); SHU, Xingtian, Beijing 100083 (CN)
(74) Representative: karo IP
(86) International application number: PCT/CN2023/091038
(87) International publication number: WO 2023/208085

(57) **Abstract**

A catalyst regeneration method suitable for use in a fluidized catalytic cracking unit is disclosed. The fluidized catalytic cracking unit includes a catalytic cracking reactor and a catalyst regenerator. The regeneration method comprises the steps of: 1) providing a bio-based liquid phase fuel; 2) introducing the bio-based liquid phase fuel into a catalyst regenerator or a stripping section of a catalytic cracking reactor; 3) introducing an oxygen-containing gas into the catalyst regenerator, wherein the oxygen-containing gas has an oxygen content of from 14 to 28 volume%; and 4) sending the spent catalyst from the catalytic cracking reactor to a catalyst regenerator, where the spent catalyst is contacted with the bio-based liquid phase fuel or the residue thereof and oxygen-containing gas to carry out coke burning regeneration. On one hand, the method can greatly reduce the carbon emission of the catalytic cracking unit and can also provide energy for other process units; on the other hand, part of the bio-based liquid phase fuel can be converted into chemicals, so that the green and low-carbon development of the catalytic cracking process is realized.

## Description

### Technical Field

The application relates to regeneration of a carbon-containing catalytic cracking catalyst, in particular to a catalytic cracking catalyst regeneration method and system adopting a bio-based liquid phase fuel.

### Background

At present, the development of the global oil refining industry faces a plurality of challenges such as new energy substitution, increasingly stringent requirements on energy conservation and emission reduction, and the like. The flexible adjustment of the cracking production process, the reduction of carbon dioxide emission, and the alleviation of climate change becomes the necessary routes for the conversion of the economic growth mode and the maintenance of sustainable development in the oil refining industry. There is urgency to achieve carbon peaking in 2030 and carbon neutralization in 2060. The 14th Five-Year Plan establishes a carbon peak-reaching action scheme, clearly requires acceleration of green development, and the China's National Emissions Trading System is formally started in 2021. Therefore, it is essential to effectively reduce carbon emissions in petroleum refining and chemical production processes, and the research on a low-carbon catalytic cracking process for oil reduction and chemical increase is an important task in the future of refineries. The carbon emission in the heavy oil processing process mainly comprises the flue gas emission of equipment such as catalytic cracking coke burning, hydrogen production process, boilers, and the like, and the energy consumption of the technological process. The catalytic cracking unit is a core device in a refinery. Carbon emission caused by coke burning of the catalytic cracking regenerator accounts for 24-55% of the carbon emission of the whole refinery, and accounts for nearly 1% of the total carbon dioxide emission of the whole country. It is a key point in carbon emission reduction in the petrochemical industry.

On the other hand, biomass oil produced from biomass or bio-based liquid fuels such as crude glycerin, which is a byproduct of the biodiesel industry, is complex in composition. Therefore, it is difficult to utilize them directly, and the purification and separation will further increase the cost. Effectively developing and utilizing biological resources has become an urgent problem to be solved.

CN1600431A discloses an incomplete regenerated flue gas combustion technology, which adopts a method of supplementing air in the incomplete regenerated flue gas to make the CO in the un-regenerated flue gas continuously combust, to raise the temperature of the flue gas, improve the recovery efficiency of the flue gas, and maximize the pressure of the recovered flue gas, thereby reducing the energy consumption of the device. The method can improve energy utilization efficiency, but cannot effectively reduce carbon dioxide emissions.

US20080153689A1 discloses a system and method for reducing carbon dioxide emissions in a fluidized catalytic cracking unit. The method comprises the following steps: compressing a first gas at an inlet pressure to a predetermined high pressure to define a compressed gas and combusting a second gas with the compressed gas to a predetermined temperature to define a heated gas; expanding the heated gas to a predetermined low pressure to define a feed gas; introducing a raw material gas into a regenerator; the raw material gas at high temperature provides heat to the regenerator to burn off coke from the waste catalyst in the reactor to achieve a certain proportion of carbon monoxide in the flue gas. This reduces the concentration of carbon dioxide in the flue gas.

TW201317354A discloses a method for synthesizing polyhydroxyalkanoate by using crude glycerol as raw material and using microorganisms, which comprises injecting crude glycerol into a fermentation tank containing bacterial strain and fermenting under certain conditions to obtain polyhydroxyalkanoate. However, the product of this process needs to be extracted and separated. Additionally, the production efficiency is low. Therefore, the large-scale industrial application is limited.

The reaction-regeneration system of the catalytic cracking unit is a periodic heat extraction-heat release heat balance process. The heat generated by the coke burning of the regeneration system is supplied for the reaction system. The production mode of oil reduction and chemical increase is beneficial to promoting the sustainable development of the oil refining industry, but more reaction heat is also needed. When the burning amount is not enough to meet the energy consumption of the device, additional fossil fuel is usually added for heat supplement, so that the emission of carbon dioxide is increased, and the resource is wasted. There is a conflict between the development trend and the environmental protection requirement. The optimization of the regeneration process or the recycling of the discharged carbon dioxide can also reduce the discharge amount of the carbon dioxide to a certain degree. However, the above technical route mainly aims at reducing the emission of carbon dioxide generated in the regeneration process into the atmosphere and does not reduce the generation of carbon dioxide. On the other hand, the bio-based derived liquid phase crude product is difficult to use directly. Its separation and purification process is complex, and the cost is high. However, the liquid phase crude product can be added as a fuel for direct combustion, without the tedious separation. This can combine well with the requirement that a catalytic cracking unit needs additional fuel for heat supplement. The bio-based fuel belongs to green zero-carbon energy and can effectively reduce the emission of carbon dioxide.

Therefore, the utilization of the bio-based derived liquid phase crude product is combined with the catalytic cracking, so that the contradiction of the development of a catalytic cracking unit can be relieved, and the utilization approach of biomass can be widened. The emission of carbon dioxide is reduced while meeting the energy supply required by the device. The low-carbon development is realized.

### Disclosure of Invention

The application aims to provide a catalyst regeneration method and a catalyst regeneration system suitable for a fluidized catalytic cracking unit, which utilizes bio-based liquid phase fuel as an energy source of the catalytic cracking unit. The catalyst regeneration method and the catalyst regeneration system can reduce carbon dioxide emissions from fossil energy while meeting the heat balance of the catalytic cracking unit. The catalyst regeneration method and the catalyst regeneration system can partially convert bio-based liquid phase fuel crude products into high-value products, and realize a high-efficiency utilization of the crude products.

To achieve the above objectives, in one aspect, the present application provides a catalyst regeneration method suitable for a fluidized catalytic cracking unit including a catalytic cracking reactor and a catalyst regenerator. The regeneration method includes the following steps:
1) Providing a bio-based liquid phase fuel;
2) Introducing the bio-based liquid phase fuel into a catalyst regenerator or a stripping section of a catalytic cracking reactor;
3) Introducing an oxygen-containing gas into the catalyst regenerator, wherein the oxygen-containing gas has an oxygen content of from 14 to 28 volume percent; and
4) Delivering the spent catalyst from the catalytic cracking reactor to a catalyst regenerator, where it is contacted with the bio-based liquid phase fuel or its residue and an oxygen-containing gas for coke-burning regeneration.

Preferably, the operating temperature of the catalyst regenerator is within the range of 550-750 °C, and the average catalyst residence time is 1.0-20.0 minutes.

In another aspect, the present application provides a catalyst regeneration system suitable for use in a fluidized catalytic cracking unit, comprising a biomass treatment unit and a catalyst regeneration unit, wherein:
The biomass treatment unit is used for the liquefaction treatment of biomass to obtain a bio-based liquid phase fuel and comprises a biomass liquefaction treatment device, a dewatering device, and a storage tank, wherein the biomass liquefaction treatment device is preferably selected from a biomass hydrolysis fermentation device, a biomass pyrolyzer, a biomass hydrothermal liquefier, and a biomass alcohol thermal liquefier, or a combination thereof, and is provided with a biomass inlet and a liquid phase product outlet. The liquid phase product outlet of the biomass liquefaction treatment device is communicated with the inlet of the dewatering device, and the outlet of the dewatering device is communicated with the inlet of the storage tank;
The catalyst regeneration unit is employed to regenerate the spent catalyst from a catalytic cracking reactor and comprises a catalyst regenerator. The catalyst regenerator includes a spent catalyst inlet, an oxygen-containing gas inlet, an optional liquid phase fuel inlet, a regenerated flue gas outlet, and a regenerated catalyst outlet, and

The outlet of the storage tank is communicated with the liquid phase fuel inlet of the catalyst regenerator or the stripping section of the catalytic cracking reactor.

Preferably, the regeneration system further includes a spent catalyst delivery sloped tube communicating the catalytic cracking reactor with a spent catalyst inlet of the catalyst regenerator, wherein:
The outlet of the storage tank is communicated with the stripping section of the catalytic cracking reactor, so that the bio-based liquid phase fuel from the storage tank enters the stripping section, and then the reaction residue of the bio-based liquid phase fuel is conveyed to the spent catalyst inlet of the catalyst regenerator together with the spent catalyst through the spent catalyst delivery sloped tube; or alternatively
A mixing tank is disposed on the spent catalyst delivery sloped tube, and an outlet of the storage tank is communicated with the mixing tank so that the bio-based liquid phase fuel from the storage tank and the spent catalyst are mixed in the mixing tank and then are conveyed to a spent catalyst inlet of the catalyst regenerator through the spent catalyst delivery sloped tube.

Compared with the existing catalytic cracking catalyst regeneration method and system, the method and system of the application have the following advantages:
(1) The bio-based liquid fuel belongs to green zero-carbon energy, wherein carbon is derived from carbon dioxide captured by plants from air, instead of fossil energy, and is a neutral carbon emission process; it is used to supply energy for a catalytic cracking unit, so that the consumption of fossil energy and the emission of carbon dioxide in the operation process can be reduced, and the low-carbon development of oil refining is realized;
(2) The bio-based liquid fuel is easy to obtain and can be obtained by liquefaction of biomass. The biomass resource storage capacity is large, and the liquefaction treatment is simple. The bio-based liquid fuel can also be derived from byproducts of industries such as biodiesel and the like;
(3) The bio-based liquid fuel can be introduced into a catalytic cracking unit without separation or purification and is coupled with a catalytic cracking process so that the biomass utilization process is simplified, the cost is saved, and the utilization mode and the way of biomass energy are expanded;
(4) The bio-based liquid fuel is introduced into the catalytic cracking unit, can only supply energy to the device, can also be partially converted into chemicals, improves the economic value, and does not cause adverse effects on the original catalytic cracking reaction process;
(5) The bio-based liquid phase fuel has a higher hydrogen content and can generate a large amount of water steam in the combustion process. Therefore, on one hand, the water steam can be utilized to age the catalyst by regulating and controlling the operation conditions of the regeneration process, so that the selectivity of the target low-carbon olefin product is improved; on the other hand, when the catalyst is required to have high activity, the adverse effect of the water steam on the activity of the catalyst can be weakened through the optimization of the regeneration process, so that the high activity of the catalyst is maintained;
(6) The excess heat generated by the regeneration system can be used for generating high-pressure steam to supply other devices. After the generated flue gas is subjected to energy recovery, the carbon dioxide in the flue gas can be separated and trapped, so that a negative carbon emission is realized.

Additional features and advantages of the application will be set forth in the detailed description which follows.

### Drawings

The accompanying drawings, which can provide a further understanding of the application and constitute a part of this specification, illustrate the application together with the detailed description below and not to limit the application. In the drawings:
Fig. 1 is a schematic diagram of a preferred embodiment of the catalyst regeneration method and system of the present application;
Fig. 2 is a schematic diagram of another preferred embodiment of the catalyst regeneration method and system of the present application; and
Fig. 3 is a schematic diagram of yet another preferred embodiment of the catalyst regeneration method and system of the present application.

### Detailed Description

The following describes in detail the embodiments of the present application with reference to the drawings. It should be understood that the specific embodiments described herein are only used to illustrate and explain the present application, and are not used to limit the present application.

Herein, the expression "exemplary" means "serving as an embodiment, example, or illustration". Any embodiment described herein as "exemplary" is not necessarily to be construed as preferred or advantageous over other embodiments. While the various aspects of the embodiments are presented in drawings, the drawings are not necessarily drawn to scale unless specifically indicated.

Any specific value disclosed herein (including endpoints of ranges of values) is not to be limited to the precise value of that value, and is to be understood to also encompass values close to the precise value, for example, all possible values within ±5% of the precise value. Also, for the disclosed ranges of values, any combination between the end points of the range, between the endpoints and the specific points within the range, and between the specific points can result in one or more new ranges of values, and such new ranges of values should also be considered to be specifically disclosed herein.

In the present application, the terms "upstream" and "downstream" are used in terms of the direction of flow of the reaction material. For example, when the reactant stream flows from bottom to top, "upstream" refers to a position below, and "downstream" refers to a position above.

In the present application, it should be noted that the directions or positional relationships indicated by the terms "above", "below", "internal", "external", "front", "back", "left", "right", etc. are directions or positional relationships based on the working state of the present application. They are only for the convenience of describing the present application and simplifying the description. They do not indicate or imply that the device or element referred to must have a specific direction, or be constructed and operated in a specific direction. Therefore, they should not be understood as limitations on the present application.

In the present application, it should be noted that the terms "mounted," "connected," "linked", and "communicating" are to be construed broadly, unless otherwise explicitly specified or limited. The specific meanings of the above terms in the present application can be understood according to specific situations by those of ordinary skill in the art. For example, in the present application, the term "communicate" includes both a case where both are in direct communication and a case where both are in communication via one or more intermediate devices.

Unless otherwise defined, terms used herein have the same meaning as commonly understood by one of ordinary skill in the art, and if a term is defined herein and its definition is different from the definition commonly understood in the art, the definition herein controls.

In the present application, anything, which are not mentioned, are directly applicable to those known in the art without any change, except what is explicitly stated. Moreover, any embodiment described herein may be freely combined with one or more other embodiments described herein, and the technical solutions or concepts formed thereby are considered part of the original disclosure or original description of the present application, and should not be considered as new matters not disclosed or contemplated herein, unless such combination is considered clearly unreasonable by those skilled in the art.

All patent and non-patent documents referred to herein, including but not limited to textbooks and journal articles and the like, are incorporated into herein by reference in their entirety.

In addition, the technical features involved in the different embodiments of the present application described below may be combined with each other as long as they do not conflict with each other.

As described above, in the first aspect, the present application provides a catalyst regeneration method suitable for a fluidized catalytic cracking unit comprising a catalytic cracking reactor and a catalyst regenerator, the regeneration method includes the steps:
1) Providing a bio-based liquid phase fuel;
2) Introducing the bio-based liquid phase fuel into a catalyst regenerator or a stripping section of a catalytic cracking reactor;
3) Introducing an oxygen-containing gas into the catalyst regenerator, wherein the oxygen-containing gas has an oxygen content of from 14 to 28 volume percent; and
4) Sending spent catalyst from the catalytic cracking reactor to a catalyst regenerator where it is contacted with the bio-based liquid phase fuel or its residue and an oxygen-containing gas for coke-burning regeneration.

In a preferred embodiment, the operating temperature of the catalyst regenerator is in the range of 550-750°C, and the average catalyst residence time is 1.0-20.0 minutes.

In the present application, the term "bio-based liquid phase fuel" refers to liquid phase fuels derived from various biomass sources, such as liquid phase products obtained by liquefaction treatment of biomass and bio-based crude glycerol produced as a by-product of industries such as biodiesel, and the like.

In some specific embodiments, the bio-based liquid phase fuel is a liquid phase product obtained by liquefaction treatment of biomass. The liquefaction treatment of the biomass may be hydrolytic fermentation, pyrolysis, hydrothermal liquefaction and/or alcohol thermal liquefaction, and the bio-based liquid phase fuel may be an alcohol-based fuel and/or a biomass oil. The crude bio-based liquid fuel product obtained by liquefaction of the biomass only needs to be dehydrated and does not need to go through other separation and purification processes before it can be used in the method of the present application, thereby achieving efficient utilization of the bio-based liquid fuel.

In other specific embodiments, the bio-based liquid phase fuel is crude glycerol, which is a byproduct of the biodiesel industry, the grease saponification industry, the fatty alcohol industry, and the like; the bio-based crude glycerol mainly comprises 10-90 wt.% of glycerol, 1-30 wt.% of methanol, 1-30 wt.% of fatty acid (ester), fat and the like, and the balance of water.

The utilization of biomass is an indirect solar energy utilization process in essence. Carbon in biomass comes from carbon dioxide captured by plants from the atmosphere, and energy consumed by the whole process also comes from solar energy. Therefore, the utilization of biomass energy is also a recycling of carbon elements and is a neutral discharge process of carbon. The zero-carbon energy bio-based liquid fuel is used as a power source of the catalytic cracking unit, so that the use of fossil energy in the operation process of the unit can be reduced, resources are saved, carbon emission reduction is realized, and the contradiction between the increasing demand for energy supply from catalytic cracking and the environmental protection requirement is relieved. The injected bio-based liquid fuel can be partially converted into chemicals, and has no influence on the original catalytic cracking process, so that a path is provided for efficiently utilizing the biomass oil. The regeneration process can also adopt pure oxygen regeneration, so that the regenerated flue gas only contains carbon dioxide and oxygen, the separation and trapping cost is reduced, and the negative carbon emission can be realized.

According to the present application, in a preferred embodiment, the biomass includes but is not limited to agricultural and forestry biomass, forestry biomass, aquatic plants, energy economic crops, waste, domestic sewage and industrial organic sewage, etc. For example, agricultural and forestry biomass includes but is not limited to straw, husk, cotton stalks, etc., the forestry biomass includes but is not limited to firewood, fast-growing forests, forestry processing residues, etc., the aquatic plants include but are not limited to reeds, algae, etc., the energy economic crops include cassava, canola etc., the waste includes but is not limited to waste paper, and the domestic sewage and industrial organic sewage include but are not limited to cooling water, kitchen drainage, organic sewage discharged from brewing, food and other industries.

In a preferred embodiment, the ratio between the amount of spent catalyst and the amount of bio-based liquid phase fuel introduced in step 2) is 5-400:1 in mass ratio.

In a preferred embodiment, the oxygen-containing gas is selected from air and diluted oxygen, which is diluted by recycled flue gas
In certain preferred embodiments, in step 2), the bio-based liquid phase fuel is directly injected into the regenerator through a distributor or into a mixing tank disposed on a spent catalyst delivery sloped tube for transporting the spent catalyst. The bio-based liquid phase fuel is pre-mixed with the spent catalyst from the catalytic cracking reactor in the mixing tank and then fed into the catalyst regenerator together with the spent catalyst. In this embodiment, it is beneficial to evenly mix the spent catalyst with the liquid fuel, thereby avoiding the occurrence of local hot spots in the regenerator bed that may damage the catalyst performance.

In other preferred embodiments, in step 2), the bio-based liquid phase fuel is injected into the stripping section of the catalytic cracking reactor to make it contact and react with the coked catalyst. The residual part after the reaction is sent to the catalyst regenerator along with the spent catalyst. In this embodiment, a portion of the liquid phase fuel can be converted into a high-value chemical, such as propylene, by reacting the bio-based liquid phase fuel with the coked catalyst. The high-value chemical, such as propylene, enters the product fractionation unit together with the catalytic cracking products to be recovered as products, thereby achieving full and effective utilization of bio-based liquid phase fuel.

In certain preferred embodiments, the catalyst regenerator is a single-stage regenerator, and the operating conditions of the regenerator include a temperature of 570-750 °C, an average catalyst residence time of 3-20 minutes, and a superficial linear velocity of the gas of 0.4-1.8 m/s.

Since the bio-based liquid phase fuel produced by biomass liquefaction has a high hydrogen content, a large amount of water steam will be generated when it burns in the catalyst regenerator. To avoid the adverse effects of the water steam on the activity of the catalyst, the method of the present application is preferably carried out in a two-stage regenerator or a double regenerator. The liquid phase fuel is only fed into the coke-burning section of the two-stage regenerator or the first regenerator of the double regenerator or is simultaneously fed into the coke-burning section and regeneration section of the two-stage regenerator or the first regenerator and the second regenerator of the double regenerator, preferably only fed into the coke-burning section or the first regenerator, and the operating conditions in the two-stage regenerator and the double regenerator are appropriately optimized.

In other preferred embodiments, the catalyst regenerator is a two-stage regenerator comprising a coke-burning section and a regeneration section in fluid communication. In step 2), the bio-based liquid phase fuel is introduced into the stripping stage of the catalytic cracking reactor or the coke-burning and/or regeneration section of the regenerator. In step 3), an oxygen-containing gas is introduced into the bottoms of the coke-burning and regeneration sections, respectively. In step 4), the spent catalyst is sent to the coke-burning section. Further preferably, the operating conditions of the coke-burning section include an operation temperature of 560 °C-720 °C, an average catalyst residence time of 10-150 seconds, and a superficial linear velocity of the gas of 0.8-3.0 m/s; and the operating conditions of the regeneration section include an operation temperature of 580-750 °C, an average catalyst residence time of 1.0-5.0 minutes, and a superficial linear velocity of the gas of 0.3-0.8 m/s. Still further preferably, the operating temperature of the regeneration section is 10-150 °C higher than the operating temperature of the coke-burning section.

In certain further preferred embodiments, the operating conditions of the coke-burning section include: 600-700 °C, with an average catalyst residence time of 60.0-120.0 seconds; and the operating conditions of the regeneration section include: 650-720 °C, with an average catalyst residence time of 2.0-4.0 minutes so that the water steam generated by the combustion of the liquid phase fuel can be used for aging the catalyst in the regeneration process of the catalyst, thereby improving the selectivity of the catalyst on the target low-carbon olefin product, avoiding the excessive influence of the water steam on the activity of the catalyst, and being suitable for the fluidized catalytic cracking process mainly for producing the low-carbon olefin. In such preferred embodiments, the liquid phase fuel can be injected into only the coke-burning section of the regenerator, or into both the coke-burning section and regeneration section of the regenerator.

In yet other further preferred embodiments, the operating conditions of the coke-burning section include 560 °C-650 °C, with an average catalyst residence time of 10.0 to 60.0 seconds; and the operating conditions of the regeneration section include 600 °C to 680 °C, with an average catalyst residence time of 1.0 to 3.0 minutes, thereby fully weakening the adverse effect of the water steam on the activity of the catalyst, so that the obtained regenerated catalyst may have higher catalytic cracking activity, and be suitable for the fluidized catalytic cracking which mainly produces fuel oil. In such preferred embodiments, the liquid phase fuel is injected only into the coke-burning section of the regenerator.

In other preferred embodiments, the catalyst regenerator is a double regenerator comprising a first regenerator and a second regenerator in fluid communication. In step 2) the bio-based liquid phase fuel is introduced into the stripping section of the catalytic cracking reactor, the first regenerator and/or the second regenerator. In step 3) an oxygen-containing gas is introduced into the bottom of the first regenerator and the bottom of the second regenerator respectively. In step 4) the spent catalyst is sent to the first regenerator. Further preferably, the operating conditions of the first regenerator include an operation temperature of 550-720 °C, an average catalyst residence time of 1.0-5.0 min, and a superficial linear velocity of the gas of 0.4-1.0 m/s; and the operating conditions of the second regenerator include an operation temperature of 570-750 °C, an average catalyst residence time of 1.0-10.0 minutes, and a superficial linear velocity of the gas of 0.3-0.8 m/s. Still further preferably, the operating temperature of the second regenerator is 10-150 °C higher than the operating temperature of the first regenerator.

In certain further preferred embodiments, the operating conditions of the first regenerator include: 600-700 °C, with an average catalyst residence time of 2.0-5.0 min; and the operating conditions of the second regenerator include: 650-720 °C, with an average catalyst residence time of 3.0-7.0 minutes, so that the water steam generated by the combustion of the gas-phase fuel can be utilized to age the catalyst in the regeneration process of the catalyst, thereby improving the selectivity of the catalyst on the target low-carbon olefin product, avoiding the excessive influence of the water steam on the activity of the catalyst, and being suitable for the fluidized catalytic cracking process mainly for producing chemicals such as low-carbon olefin. In such preferred embodiments, the liquid phase fuel may be injected into only the first regenerator or into both the first and second regenerators.

In yet other further preferred embodiments the operating conditions of the first regenerator include 550 °C-650 °C, with an average catalyst residence time of 1.0-4.0 min; and the operating conditions of the second regenerator include 600 °C to 680 °C, with an average catalyst residence time of 2.0 to 5.0 minutes, thereby fully weakening the adverse effect of the water steam on the activity of the catalyst, so that the obtained regenerated catalyst may have higher catalytic cracking activity, and be suitable for the fluidized catalytic cracking mainly for producing fuel oil. In such preferred embodiments, the liquid phase fuel is injected only into the first regenerator.

In certain preferred embodiments, step 1) further comprises subjecting biomass to a liquefaction treatment to obtain the bio-based liquid phase fuel, wherein the liquefaction treatment is selected from the group consisting of hydrolytic fermentation, pyrolysis, hydrothermal liquefaction, and alcohol thermal liquefaction. The bio-based liquid phase fuel is selected from the group consisting of alcohol-based fuels and biomass oil.

In certain further preferred embodiments, step 1) comprises subjecting the biomass to acid hydrolysis or enzymatic hydrolysis and then subjecting the resulting hydrolysate to microbial fermentation to yield an aqueous alcohol-based fuel. The liquid phase product obtained by fermentation is dewatered to obtain the alcohol-based fuel, and the alcohol-based fuel can be directly used as the liquid phase fuel of the present application without separation. Further preferably, the temperature of the acid hydrolysis is not more than 200 °C, the temperature of the enzyme hydrolysis is not more than 70 °C, the fermentation temperature is not more than 50 °C, is preferably 35-50 °C, and the microorganism is selected from bacteria, fungi, and yeast.

According to the application, the energy consumed by the process of preparing the alcohol fuel from the biomass can be at least partially or completely from renewable energy sources such as solar energy, green electricity, nuclear energy, and the like.

According to the application, the alcohol-based fuel mainly comprises ethanol and also comprises a small amount of methanol, saturated monohydric alcohols with 3-5 carbon atoms, organic acids, ethers, esters, ketones, aldehydes and the like. In one embodiment, the alcohol-based fuel comprises greater than 90% ethanol, no greater than 5% water, and the balance of methanol and C₃₋₅ saturated monohydric alcohols, based on the total weight of the alcohol-based fuel.

In yet further preferred embodiments, step 1) comprises subjecting the biomass to fast pyrolysis or flash pyrolysis at a heating rate of 100-200°C/s with a residence time of 2-10s to obtain biomass oil.

In still other further preferred embodiments, step 1) comprises subjecting the biomass to hydrothermal liquefaction or alcohol thermal liquefaction at a treating temperature of 200-350 °C and a pressure of 4.0-7.0MPa, wherein the solvent of the alcohol thermal liquefaction is selected from methanol and ethylene glycol to obtain the biomass oil.

According to the application, biomass liquefaction (such as pyrolysis, hydrothermal liquefaction, or alcohol thermal liquefaction) is carried out to obtain a biomass oil crude product. The biomass oil obtained after moisture removal can be directly used as the liquid phase fuel of the application, wherein the biomass oil is a mixture and comprises acids, aldehydes, ketones, alcohols, esters, ethers, phenols, saccharides, oligomers, and the like.

According to the application, the energy consumed by the biomass liquefaction treatment is at least partially or completely derived from renewable energy sources such as solar energy, green electricity, nuclear energy and the like.

In some embodiments, the biomass oil is directly introduced into the mixing tank without separation, and is mixed evenly with the catalyst through the nozzle, and then is conveyed to the regenerator to contact with the catalyst and generate a combustion reaction. Preferably, the ratio of spent catalyst to biomass oil introduced is 10-300:1 (by weight).

In certain preferred embodiments, the step 1) further comprises, prior to the liquefaction treatment, pretreating the biomass to increase the efficiency of the hydrolysis or liquefaction treatment. The pretreatment method can be selected from several types such as physical pretreatment, chemical pretreatment, biological pretreatment, physical and chemical pretreatment, and the like, and the specific method is selected from one or more of crushing, drying, baking, compression molding, ball milling treatment, microwave treatment, acid treatment, alkali treatment, steam explosion (steam explosion for short), carbon dioxide explosion, microbial degradation, and the like.

In certain preferred embodiments, in step 2), the bio-based liquid phase fuel is introduced into a catalyst regenerator or a stripping section of a catalytic cracking reactor along with other sources of bio-based products, which may be by-product crude glycerol from the biodiesel industry, the grease saponification industry, the fatty alcohol industry, and the like. Preferably, the crude glycerol comprises 10-90 wt.% glycerol, 1-30 wt.% methanol, 1-30 wt.% fatty acids or fatty acid esters, and the balance of water.

In a preferred embodiment, in step 4) the temperature of the regenerator is controlled by a heat extraction system comprising one or more internal and/or external heat extractors so that it does not exceed 750 °C, preferably exceeding 720 °C. Further preferably, the heat extraction system uses heat extracted from the regenerator to generate high-pressure steam to supply energy to the outside.

The injection mode of the bio-based liquid phase fuel can be well coupled with the catalytic cracking process. The bio-based liquid phase fuel is sprayed on the spent catalyst for premixing so that the bio-based liquid phase fuel can be preheated on one hand, the local concentration can be prevented from being too high and the condition of local overheating in the coke-burning process is avoided on the other hand. The pure oxygen regeneration process only enables the regenerated flue gas to contain carbon dioxide and oxygen only, thereby reducing the separation and trapping cost and realizing the negative carbon emission. The bio-based liquid phase fuel is injected into the stripping section, so that part of the bio-based liquid phase fuel can be converted into high-value chemicals, and the rest part of the bio-based liquid phase fuel is used for supplying energy, so that the economic efficiency is improved, and the original catalytic cracking reaction process is not influenced.

In certain preferred embodiments, the temperature within the catalyst regenerator is controlled to not exceed 750 °C by a heat extraction system comprising one or more internal or/and external heat extractors. Further preferably, the heat extraction system uses the heat extracted from the catalyst regenerator to generate high-pressure steam, which is exported to other units for energy supplement. In such preferred embodiments, the present application can use the energy generated by the regeneration system of the catalytic cracking unit to supply other operation units to become the power center of the refinery, thereby fundamentally reducing the carbon emission of the refinery. According to the application, biomass is introduced into a power center of the catalytic cracking unit, the unit operation is supplied with biomass energy, and the discharged carbon dioxide is not from fossil energy, so that the energy source can be fundamentally changed, and carbon emission reduction is realized.

In a second aspect, the present application provides a catalyst regeneration system suitable for use in a fluidized catalytic cracking unit, wherein the catalyst regeneration system includes a biomass treatment unit and a catalyst regeneration unit, wherein:
the biomass treatment unit is used for liquefying biomass to obtain a bio-based liquid phase fuel and comprises a biomass liquefaction treatment device, a dewatering device, and a storage tank, wherein the biomass liquefaction treatment device is preferably selected from a biomass hydrolysis fermentation device, a biomass pyrolyzer, a biomass hydrothermal liquefier and a biomass alcohol thermal liquefier, or a combination thereof, and is provided with a biomass inlet and a liquid phase product outlet, the liquid phase product outlet of the biomass liquefaction treatment device is communicated with the inlet of the dewatering device, and the outlet of the dewatering device is communicated with the inlet of the storage tank;
the catalyst regeneration unit is used for regenerating spent catalyst from a catalytic cracking reactor and comprises a catalyst regenerator having a spent catalyst inlet, an oxygen-containing gas inlet, an optional liquid phase fuel inlet, a regenerated flue gas outlet, and a regenerated catalyst outlet, and
the outlet of the storage tank is communicated with the liquid phase fuel inlet of the catalyst regenerator or the stripping section of the catalytic cracking reactor.

In certain preferred embodiments, the biomass treatment unit further includes biomass pretreatment equipment for pretreating the biomass, the pretreating selected from one or more of crushing, drying, torrefaction, compression molding, ball milling, microwave treatment, acid treatment, alkali treatment, steam explosion, carbon dioxide explosion, and microbial degradation.

In certain preferred embodiments, the regeneration system further includes a spent catalyst delivery sloped tube communicating the catalytic cracking reactor with a spent catalyst inlet of the catalyst regenerator, wherein an outlet of the storage tank is communicated with a stripping section of the catalytic cracking reactor, so that the bio-based liquid phase fuel from the storage tank enters the stripping section, and then reaction residues thereof are conveyed along with the spent catalyst to the spent catalyst inlet of the catalyst regenerator through the spent catalyst delivery sloped tube.

In other preferred embodiments, the regeneration system further comprises a spent catalyst delivery sloped tube communicating the catalytic cracking reactor with a spent catalyst inlet of the catalyst regenerator. A mixing tank is disposed on the spent catalyst delivery sloped tube, and an outlet of the storage tank is communicated with the mixing tank so that the bio-based liquid phase fuel from the storage tank and the spent catalyst are mixed in the mixing tank and then conveyed to the spent catalyst inlet of the catalyst regenerator through the spent catalyst delivery sloped tube.

In other preferred embodiments, a liquid phase fuel distributor is further provided at the liquid phase fuel inlet of the catalyst regenerator for directly injecting the liquid phase fuel into the regenerator.

In certain preferred embodiments, the catalyst regenerator comprises a coke-burning section and a dense phase regeneration section. The dense phase regeneration section is located above the coke-burning section and the outlet of the coke-burning section is contained within the dense phase regeneration section such that the coke-burning section is in fluid communication with the dense phase regeneration section;

The burning section is provided with:
a first oxygen-containing gas inlet, which is disposed at the bottom of the coke-burning section for inputting oxygen-containing gas into the coke-burning section;
an optional first liquid phase fuel inlet and an optional first liquid phase fuel distributor, which is disposed above the first oxygen-containing gas inlet for direct input of the bio-based liquid phase fuel from the storage tank into the coke-burning section;
the spent catalyst inlet, which is used for conveying spent catalyst from a catalytic cracking reactor to the interior of the coke-burning section; and
an optional first circulating flue gas inlet, which is employed for circulating a portion of the flue gas exited from the dense phase regeneration section back into the interior of the coke-burning section.

The dense phase regeneration section is provided with:
a second oxygen-containing gas inlet, which is disposed at the bottom of the dense phase regeneration section, and is used for introducing an oxygen-containing gas into the dense phase regeneration section;
an optional second liquid phase fuel inlet and an optional second liquid phase fuel distributor, which is disposed above the second oxygen-containing gas inlet, and is used for directing the bio-based liquid phase fuel from the storage tank into the dense phase regeneration section;
the regenerated flue gas outlet, which is disposed at the top of the dense phase regeneration section, and is used for discharging the regenerated flue gas in the dense phase regeneration section;
the regenerated catalyst outlet, which is used for returning the regenerated catalyst to the catalytic cracking reactor; and
an optional second circulating flue gas inlet, which is used for circulating a portion of the flue gas discharged from the dense phase regeneration section back into the interior of said dense phase regeneration section.

In a further preferred embodiment, the dense phase regeneration section is further disposed with a heat extraction system including one or more internal and/or external heat extractors for controlling the temperature of the dense phase regeneration section and optionally for generating high-pressure steam to supply energy to the outside.

In other preferred embodiments, the catalyst regenerator comprises a first regenerator and a second regenerator. The second regenerator is located downstream of the first regenerator. The first and second regenerators are connected by a catalyst transfer line, the catalyst material partially regenerated by the first regenerator is transferred to the second regenerator.

The first regenerator is provided with:
a first oxygen-containing gas inlet, which is disposed at the bottom of the first regenerator, and is used for inputting an oxygen-containing gas to the first regenerator;
an optional first liquid phase fuel inlet and an optional first liquid phase fuel distributor, which is disposed above the first oxygen-containing gas inlet, and is used for feeding the bio-based liquid phase fuel from the storage tank directly into a first regenerator;
the spent catalyst inlet, which is used for conveying the spent catalyst from the catalytic cracking reactor to the interior of the first regenerator; and
a first regenerated flue gas outlet, which is disposed at the top of the first regenerator, and is used for discharging the regenerated flue gas in the first regenerator.

The second regenerator is provided with:
a second oxygen-containing gas inlet, which is disposed at the bottom of the second regenerator, and is used for inputting oxygen-containing gas to the second regenerator;
an optional second liquid phase fuel inlet and an optional second liquid phase fuel distributor, which is disposed above the second oxygen-containing gas inlet, and is used for feeding the bio-based liquid phase fuel from the storage tank directly into a second regenerator;
the regenerated catalyst outlet, which is used for returning the regenerated catalyst to the catalytic cracking reactor; and
a second regenerated flue gas outlet, which is disposed at the top of the second regenerator, and is used for discharging the regenerated flue gas in the second regenerator.

In a further preferred embodiment, the first and second regenerators are further provided with a heat extraction system comprising one or more internal and/or external heat extractors, for controlling the temperature of the first and second regenerators and optionally for generating high-pressure steam to effect external power supply.

The preferred embodiment of the regeneration method and system of the present application will be further described in detail below in conjunction with the accompanying drawings.

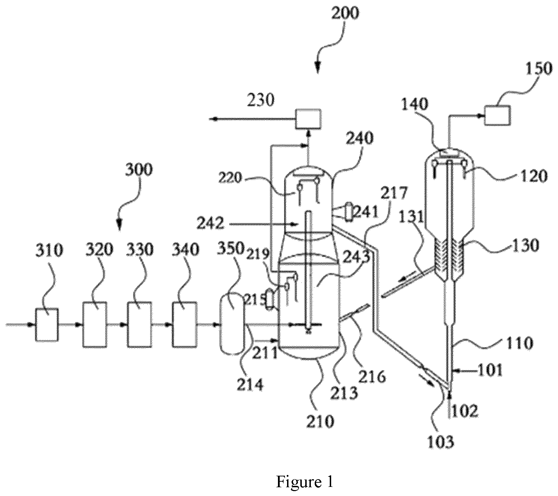

As shown in Fig. 1, 2 and 3, the catalyst regeneration system of the present application is adapted to regenerate a spent catalyst from a catalytic cracking reaction unit 100, and includes biomass treatment units 300 and 500, and catalyst regeneration units 200, 400, and 600.

As shown in Fig. 1, 2 and 3, in the catalytic cracking reaction unit 100, the catalytic cracking reactor 110 serves to perform a catalytic cracking reaction: a lifting medium is fed through its bottom inlet 102 to lift the regenerated catalyst (from the regenerator) entering through the regenerated catalyst inlet 103; the raw material oil entering from the raw material oil inlet 101 contacts the catalyst to carry out a catalytic cracking reaction. The oil and gas products of the reaction are separated by the oil agent separation device 120. The separated oil and gas products are gathered in the gas collection chamber 140 and then delivered into the product separation device 150 for separation, to obtain various products. The separated spent catalyst is transported to the regeneration unit through the stripping section 130 of the settler and the spent catalyst outlet 131 for regeneration, thereby realizing recycling. The catalytic cracking reactor 110 suitable for the present application can be various reactors commonly used in the art, such as a riser reactor, a fluidized bed reactor, a variable diameter reactor, and a combination thereof.

The biomass treatment unit 300 shown in Fig. 1 includes:
a biomass pre-treatment equipment 310 for biomass pretreatment, and
the biomass hydrolysis device 320 for carrying out hydrolysis treatment on the pretreated biomass to obtain hydrolysate,
a hydrolysate fermentation device 330 for performing fermentation treatment on the hydrolysate to obtain a liquid phase product;
the dehydration device 340 for dehydrating the liquid phase product to obtain the alcohol-based fuel; and
a storage tank 350 for storing the alcohol-based fuel.

As shown in the figure, the biomass can be delivered to a pretreatment unit 310. After pretreatment, it is delivered to a hydrolysis device 320 to obtain a hydrolysate. The hydrolysate is transported to a fermentation device 330, and the liquid phase product obtained by fermentation is transported to the dehydration device 340 to remove water, and transported to the storage tank 350 for the subsequent regeneration treatment process.

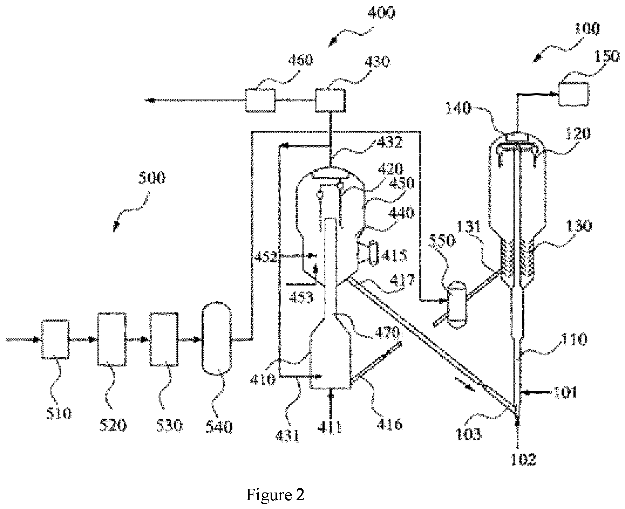

The biomass treatment unit 500 shown in Fig. 2 includes:
a biomass pre-treatment equipment 510 for biomass pretreatment,
a biomass liquefaction treatment device 520 for processing the pretreated biomass to obtain a biomass oil crude product,
a dehydration system 530 for performing dehydration treatment on the crude biomass oil product to obtain biomass oil; and
a storage tank 540 for storing the biomass oil.

Fig. 1 shows a schematic diagram of the first preferred embodiment of the catalyst regeneration method and system of the present application. As shown in Fig. 1, the catalyst regeneration unit 200 comprises a first regenerator 210, a first heat extractor 215, a second regenerator 240, and a second heat extractor 241, wherein the first regenerator 210 and the second regenerator 240 are in communication with each other such that the material of the first regenerator can enter the second regenerator. The first heat extractor 215 is configured to extract excess heat from the first regenerator for delivery to an external device. The second heat extractor 241 is configured to extract excess heat from the second regenerator for delivery to an external device.

In one embodiment, the first regenerator 210 is located below the second regenerator 240 and is in communication with the second regenerator 240 via a riser 243. The riser 243 has one end opening at the internal of the first regenerator 210 and the other end opening at the internal of the second regenerator 240 so that the first regenerator 210 and the second regenerator 240 are communicated with each other through the riser 243. Thereby the material in the first regenerator 210 can enter the second regenerator 240 through the riser 243.

The first regenerator 210 is provided with:
an alcohol-based fuel inlet 214 for inputting alcohol-based fuel from the storage tank to the first regenerator;
a distributor 213 configured to distribute the alcohol-based fuel input through the alcohol-based fuel inlet;
a first oxygen-containing gas inlet 211, which is provided at the bottom of the first regenerator, and is used for inputting the first oxygen-containing gas to the first regenerator; and
a spent catalyst inlet 216 for delivering spent catalyst from the catalytic cracking reactor to the interior of the first regenerator;
wherein the storage tank 350 is in communication with the alcohol-based fuel inlet 214 such that the alcohol-based fuel is delivered to the interior of the first regenerator 210.

At the internal of the first regenerator 210, an alcohol-based fuel is introduced through the spent catalyst inlet 216 and the distributor 213 and mixed with the spent catalyst. The spent catalyst completes a partial regeneration at the internal of the first regenerator in the presence of a first oxygen-containing gas. In one embodiment, the operating conditions of the first regenerator include a temperature of 580 °C-720 °C, an average catalyst residence time of 1.0-5.0 minutes, and a superficial linear velocity of the gas of 0.4-1.0 m/s.

The flue gas generated in the first regenerator 210 can be separated by the first cyclone separation system 219 and then introduced into the flue gas energy recovery device 230 for energy recovery. The catalyst separated by the first cyclone separation system 219 enters the second regenerator 240 via the riser 243.

Due to the injection of alcohol-based fuels, a large amount of heat is generated during the regeneration process. If the temperature in the first regenerator is too high, the activity of the catalyst may be adversely affected. Thus, the first regenerator 210 is further configured with a heat extractor 215 for extracting excess heat from the first regenerator. The heat extractor can be an internal heat extractor (disposed at the internal of the regenerator) or/and an external heat extractor (disposed at the external of the regenerator). The heat extractors can be one or more and can use the excess energy generated by the first regenerator to supply other devices. The excess heat of the regeneration system can be used for generating high-pressure steam by a heat extractor and is output to other devices for energy supply. In one embodiment, the first regenerator bed temperature is controlled to not exceed 720 °C by the provision of a heat extractor.

The second regenerator 240 is provided with:
a second oxygen-containing gas inlet 242 provided at the bottom of the second regenerator for inputting a second oxygen-containing gas into the second regenerator; and
a regenerated catalyst outlet 217 for delivering regenerated catalyst to the catalytic cracking reactor.

Thus, the partially regenerated catalyst in the first regenerator 210 enters the second regenerator 240 through the riser to complete the regeneration. In one embodiment, the operating conditions of the second regenerator include a temperature of 570-750 °C, an average catalyst residence time of 3.0-10.0 minutes, and a superficial linear velocity of the gas of 0.3-0.8 m/s.

Optionally, an alcohol-based fuel is also introduced to the second regenerator 240. In one embodiment, the second regenerator 240 is further provided with:
a second alcohol-based fuel inlet for inputting an alcohol-based fuel from the storage tank to the second regenerator; and
a second distributor configured to distribute the alcohol-based fuel input through the second alcohol-based fuel inlet.

Likewise, a large amount of heat is generated during regeneration in the second regenerator. If the temperature in the second regenerator is too high, the activity of the catalyst may be adversely affected. The second regenerator 240 is therefore also provided with a heat extractor 241 for extracting excess heat from said second regenerator. The heat extractor can be an internal heat extractor (disposed at the internal of the regenerator) or/and an external heat extractor (disposed at the external of the regenerator). The heat extractors can be one or more, and can use the excess energy generated by the second regenerator for supplying other devices. The excess heat of the regeneration system can be used for generating high-pressure steam by a heat extractor and is output to other devices for energy supply. In one embodiment, the second regenerator bed temperature is controlled to not exceed 750 °C, e.g., not exceed 720 °C by the provision of a heat extractor.

The flue gas generated in the second regenerator 240 can be separated by the second cyclone separation system 220 and then introduced into the flue gas energy recovery device 230 for energy recovery.

In one embodiment, the alcohol-based fuel is introduced directly into the first regenerator and optionally the second regenerator through a distributor, contacts the catalyst, and undergoes a combustion reaction. The alcohol-based fuel is introduced into the first regenerator in a ratio of 60 to 100 wt.%. The distributor is selected from a nozzle and a distribution pipe. In one embodiment, the ratio of spent catalyst to alcohol-based fuel introduced is 10-400:1 (by weight).

In one embodiment, the first oxygen-containing gas and the second oxygen-containing gas are both air. Two-stage regeneration can improve the coke-burning intensity and reduce the catalyst inventory. Besides, the two-stage regeneration can weaken the influence of water steam on the catalyst in the regeneration process. In certain further preferred embodiments, the water steam can age the catalyst to increase product selectivity by further optimizing the operating conditions of the first and second regenerators.

Fig. 2 shows a schematic diagram of a second preferred embodiment of the catalyst regeneration method and system of the present application. As shown in Fig. 2, the regeneration unit 400 includes a regenerator 440, the regenerator 440 including a coke-burning section 410, and a dense phase regeneration section 450. The dense phase regeneration section 450 is located above the coke-burning section 410, and an outlet of the coke-burning section is contained at the internal of the dense phase regeneration section such that the coke-burning section is in fluid communication with the dense phase regeneration section. Thus, the dense phase regeneration section 450 is connected to the coke-burning section 410 such that material from the coke-burning section 410 can enter the dense phase regeneration section 450 for complete regeneration.

The coke-burning section 410 is provided with:
a first oxygen inlet 411, which is disposed at the bottom of the coke-burning section, and is used for inputting oxygen to said coke-burning section; and
a spent catalyst inlet 416 for conveying spent catalyst from the catalytic cracking reactor to the interior of the coke-burning section;
wherein the spent catalyst inlet 416 is connected with a spent catalyst delivery sloped tube communicated with the catalytic cracking reaction unit, and a mixing tank 550 is disposed at the spent catalyst delivery sloped tube;

The storage tank 540 is communicated with the mixing tank 550, so that the biomass oil in the storage tank is transported to the mixing tank, mixed with the spent catalyst, and then transported to the regenerator 410.Optionally, the lower part of the coke-burning section is also provided with a first circulating flue gas inlet 431, and the first circulating flue gas inlet 431 is used for circulating a part of the flue gas recovered by the regeneration section back to the interior of the coke-burning section.

The dense phase regeneration section 450 is provided with:
a second oxygen inlet 453, which is disposed at the bottom of the dense phase regeneration section, and used for inputting oxygen into the dense phase regeneration section;
a second circulating flue gas inlet 452 for circulating a portion of the flue gas recovered from the dense phase regeneration section back into the internal of the dense phase regeneration section; and
a flue gas outlet 432, disposed at the top of the dense phase regeneration section;
wherein the dense phase regeneration section is further configured with a heat extractor 415 for transporting heat to the exterior of the regenerator.

A portion of the coke-burning section 410 is located at the external of the dense phase regeneration section 450 and a portion of the coke-burning section 410 is located at the internal of the dense phase regeneration section 450. The portion of the coke-burning section 410 located at the external of the dense phase regeneration section 450 may have a larger cross-sectional area than the portion of the coke-burning section 410 located at the internal of the dense phase regeneration section 450. In one embodiment, the portion of the coke-burning section 410 located at the external of the dense phase regeneration section 450 communicates with the interior of the dense phase regeneration section 450 through a connecting tube 470. The outlet end of the connecting tube 470 is located in the interior of the dense phase regeneration section 450.

When the regeneration operation is carried out, the biomass oil in storage tank 540 is sprayed into the mixing tank 550 to mix with the spent catalyst, and then enters the coke-burning section 410 to contact with the first oxygen entering through the oxygen-containing gas inlet 411, and a part of coke-burning reaction occurs in the coke-burning section. Then it enters the dense phase regeneration section 450 for complete regeneration. At this time, pure oxygen is supplied through the oxygen inlet 452, and contacts with the partially coke-burning catalyst, thereby further regenerating and burning the catalyst and the incompletely regenerated flue gas. The regenerated catalyst, after separation in cyclone separator 420, falls back to the dense phase regeneration section and is discharged through catalyst outlet 417 and recycled back to the catalytic cracking reactor. A part of the flue gas discharged from the flue gas outlet 432 is subjected to energy recovery by the flue gas energy recovery system 430, and then is separated by the carbon dioxide separation system 460, so that carbon dioxide is captured; the other part of the flue gas discharged from the flue gas outlet 432 is recycled to the bottom of the coke-burning section and the dense-phase regeneration section.

In one embodiment, the gas input through the first oxygen inlet 411 and the second oxygen inlet 452 is both oxygen. However, oxygen, which is fed via the first oxygen inlet 411, will mix with the recycled flue gas after entering the coke-burning section to form an oxygen-carbon dioxide mixture gas. The amount of oxygen and/or recycled flue gas is controlled such that the oxygen concentration in the mixture does not exceed 28% by volume. Similarly, oxygen introduced through the second oxygen inlet 453 is mixed with the recycled flue gas, etc. after entering the regeneration section, to form an oxygen-carbon dioxide mixture gas. The amount of oxygen and/or recycled flue gas is controlled so that the oxygen concentration in the mixture is not higher than 28% by volume. The coke is burnt under this atmosphere so that the coke burning strength is improved; the inlet gas does not contain nitrogen so that the energy consumed by gas preheating can be reduced; and the concentration of carbon dioxide in the flue gas at the outlet of the regenerator is higher, which is convenient for separating and capturing carbon dioxide.

In one embodiment, the operating conditions of the coke-burning section include an operation temperature being 560-720 °C, an average catalyst residence time being 10-120 seconds, and an superficial linear velocity of the gas being 0.8-3.0 m/s.

In one embodiment, the operating conditions of the dense phase regeneration section include an operation temperature being 580-750 °C, an average catalyst residence time being 1.0-5.0 minutes, and an superficial linear velocity of the gas being 0.3-0.8 m/s.

In a specific embodiment, the coke-burning proportion in the coke-burning section is from 40 to 50%; the coke-burning proportion of the dense phase regeneration section is 50-60%. The application adopts pure oxygen for regeneration, and the regenerated flue gas only contains carbon dioxide and oxygen, thereby being convenient for separating and capturing the carbon dioxide for further conversion and utilization and realizing negative carbon emission.

Due to the injection of biomass oil, a large amount of heat is generated during the regeneration process. If the temperature within the regenerator is too high, the activity of the catalyst may be adversely affected. Therefore, the regeneration unit 400 is also provided with a heat extractor 415 for extracting excess heat from the regenerator. The heat extractor can be an internal heat extractor (disposed at the internal of the regenerator) or/and an external heat extractor (disposed at the external of the regenerator). The heat extractors can be one or more and can use the excess energy generated by the regenerator to supply other devices. The excess heat of the regeneration system can be used for generating high-pressure steam by a heat extractor and is output to other devices for energy supply. In one embodiment, the regenerator bed temperature is controlled to not exceed 750 °C, such as not exceed 720 °C, by the provision of a heat extractor.

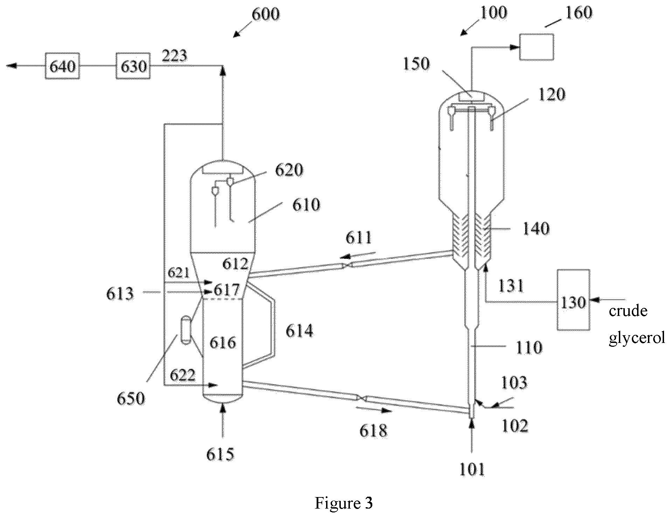

Fig. 3 shows a schematic diagram of a third preferred embodiment of the catalyst regeneration method and system of the present application. As shown in Fig. 3, the pre-lifting medium enters from the bottom of the riser reactor 110 through pipeline 101, and the regenerated catalyst from the spent catalyst delivery sloped tube 618 moves upward with the action of the pre-lifting medium. The preheated raw material oil is injected into the riser reactor through pipeline 102 together with the atomized steam from pipeline 103, contacts with the catalyst to react, and moves upward. The generated reaction product and the inactivated spent catalyst enter a cyclone separator in the settler 120 through an outlet section to realize the separation of the spent catalyst and the reaction product. The reaction product enters a gas collection chamber 150, and the catalyst returns to the settler through a dipleg. The desalted biomass oil is injected into a stripping section through pipeline 131 to contact with a spent catalyst and react, and a product and stripped oil gas enter a gas collection chamber 150 after being subjected to cyclone separation. The oil and gas in the gas collection chamber 150 pass through a large oil and gas pipeline to a subsequent product fractionation unit 160. Unreacted biomass oil and spent catalyst enter a dense-phase bed 612 of the first regenerator through a spent catalyst delivery sloped tube 611, and contact with oxygen diluted by circulating flue gas to perform a combustion reaction. The catalyst is partially regenerated, and liquid phase fuel is combusted in the first regenerator to supply power. The partially regenerated catalyst enters the second regenerator 616 through the semi-regenerated catalyst pipe 614 for complete regeneration. After cyclone separation, a part of the flue gas is circulated to the regenerator, and a part of the flue gas enters the flue gas energy recovery system 630 to recover energy and then enters the carbon dioxide separation and capture system 640. The completely regenerated catalyst is returned to the reactor through a regeneration pipeline 618 to realize cyclic utilization.

In one embodiment, the crude biomass oil product may have one or more injection ports, preferably at the bottom and/or lower portion of the stripping section. In one embodiment, the ratio of the amount of catalyst to the amount of biomass oil injected into the stripping section is 5-300:1 in mass ratio.

In one embodiment, the regeneration process injects pure oxygen and the oxygen content in the regenerator is controlled by circulating flue gas to no more than 28%.

In one embodiment, the first regenerator is operated at 550-720 °C, the average catalyst residence time is 1.0-5.0 minutes, and the superficial linear velocity of the gas is 0.4-1.0 m/s; the operation temperature of the second regenerator is 570-750 °C, the average catalyst residence time is 1.0-10.0 minutes, and the superficial linear velocity of the gas is 0.3-0.8 m/s.

In one embodiment, as the biomass oil is supplemented in the regenerator as fuel, more heat is generated to cause the temperature of the regenerator to be overhigh, the temperature of the regenerator bed is controlled not to exceed 750 °C by a heat extractor, and thereby the influence on the catalyst being prevented. The heat extractor is an inner heat extractor or/and an outer heat extractor. The heat extractors can be one or more, which is/are used for controlling the temperature of the bed layer and collecting excess heat. The excess heat of the regeneration system is used for generating high-pressure steam through the heat extractor, and the high-pressure steam is output to other devices for energy supply.

In a specific embodiment, oxygen is directly introduced for coke burning, the burning efficiency is higher, the concentration of carbon dioxide in the regenerated flue gas is higher, and the separation and the capture of the carbon dioxide in the flue gas are facilitated.

The catalyst suitable for use in the catalyst regeneration method and system of the present application may be a catalyst conventionally used in various catalytic cracking processes, and the present application is not particularly limited. In certain embodiments, the catalyst comprises a zeolite, an inorganic oxide, and optionally a clay. These components are comprised, by weight of the total catalyst, as below: 1-50 wt.% of zeolite, 5-99 wt.% of inorganic oxide and 0-70 wt.% of clay. The zeolite is an active component selected from the group consisting of mesopore zeolites and/or optionally macropore zeolites, the mesopore zeolites represent from 10 wt.% to 100 wt.% of the total weight of the zeolite, and the macropore zeolites represent from 0 wt.% to 90 wt.% of the total weight of the zeolite; the mesopore zeolite is selected from one or more of ZSM series zeolite and/or ZRP zeolite. These zeolites can be modified with nonmetals such as phosphorus and/or transition metals such as iron, cobalt, and nickel; the macropore zeolite is selected from one or more of hydrogen Y, rare earth Y, rare earth hydrogen Y, Ultrastable Y and the like.

### Examples

The present application will be further inllustrated below by way of examples, but the present application is not limited thereby.

Properties of raw material oil A used in the following examples and comparative examples are shown in Table 1.

**TABLE 1. Properties of raw material oil A**

| Properties of feed | Raw material oil A |
|---|---|
| Density, kg/cubic meter (20 °C) | 843.7 |
| C, wt.% | 86.59 |
| H, wt.% | 13.41 |
| S, µg/g | 5800 |
| N, µg/g | 62 |
| Initial distillation point, °C | 226 |
| 50% distillation temperature, °C | 287 |
| Paraffin, wt.% | 40.5 |
| Cycloalkane, wt.% | 33.3 |
| Aromatic hydrocarbons, wt.% | 26.2 |

Catalyst a is TCC, and the preparation process is as follows: 969 g of halloysite (product of China Kaolin Company, with a solid content of 73%) is pulped by 4300 g of decationized water, 781 g of pseudo-boehmite (product of Shandong Zibo Aluminum Ore Plant, with a solid content of 64%) and 144 ml of hydrochloric acid (with a concentration of 30% and a specific gravity of 1.56) were added and stirred evenly, the mixture was left to stand and aged for 1 hour at 60 °C. The pH value was kept between 2 and 4. The mixture was cooled to normal temperature. Then 5000 g of prepared high silica-alumina ratio mesopore shape-selective ZSM-5 zeolite slurry containing chemical water was added. The mixture was stirred evenly and spray-dried. Free Na⁺ was washed off. The product could be used after being aged. The aging process was as follows: the aging was carried out at 800 °C for 15h in water steam. The properties are listed in Table 2.

**TABLE 2 Properties of catalyst a**

| | |
|---|---|
| Catalyst | a |

| Chemical composition/wt.% | |
|---|---|
| Al₂O₃ | 49.2 |
| Na₂O | 0.07 |

| Physical Properties | |
|---|---|
| Specific surface area/(m²· g⁻¹) | / |
| Bulk density/(g· cm ⁻³ ) | 0.79 |
| Abrasion index/(%·h ⁻¹) | 1.1 |

| Sieving composition/wt.% | |
|---|---|
| 0-40 µm | 14.2 |
| 0-80 µm | 53.8 |
| 0-149 µm | 89.5 |

The preparation process of the catalyst b is as follows:
(1) Dissolving NH₄Cl (20 g) in 1000 g of water. 100 g (dry basis) of a crystallized product ZRP-1 molecular sieve (manufactured by Qilu Petrochemical Company Catalyst Plant, SiO₂/Al₂O₃=30, rare earth content RE₂O₃ =2.0 wt.%) was added into the solution. After exchanging at 90 °C for 0.5 hours, a filter cake was obtained after filtration. 4.0 g of H₃PO₄ (with a concentration of 85%) and 4.5 g of Fe(NO₃)₃ were added and dissolved in 90 g of water, and the filter cake was mixed, dipped, and dried with them. Then it was roasted for 2 hours at a temperature of 550 °C to obtain the MFI mesoporous molecular sieve containing phosphorus and iron. The obtained molecular sieve has the following element analysis chemical composition:

   0.1Na₂O•5.1Al₂O₃-2.4P₂O₃-1.5Fe₂O₃•3.8RE₂O₃•88.1SiO₂.
(2) 75.4 kg of halloysite (industrial product of Suzhou china Company, with a solid content of 71.6 wt.%) was slurried with 250 kg of decationized water. 54.8 kg of pseudo-boehmite (industrial product of Dong Aluminum Plant, with a solid content of 63 wt.%) was added. The pH thereof was adjusted to 2-4 with hydrochloric acid. It was stirred evenly, left to stand, and aged at 60-70 °C for 1 hour. Its pH was maintained at 2-4. Its temperature was lowered to below 60 °C. 41.5 kg of alumina sol (product of Qilu Petrochemical Company Catalyst Plant, with an Al₂O₃ content of 21.7 wt.%) was added, and it was stirred for 40 minutes to obtain a mixed slurry.
(3) The MFI mesoporous molecular sieve (2 kg dry basis) containing phosphorus and iron prepared in step (1) was added to the mixed slurry obtained in step (2). It was stirred evenly, spray-dried and formed. It was washed with ammonium dihydrogen phosphate solution (the phosphorus content is 1 wt.%). Free Na⁺ was washed away from it and it was dry to obtain the catalytic conversion catalyst sample b. Based on the total weight of the catalyst b on a dry basis, the composition of the catalyst b on a dry basis comprises 2 wt.% of MFI mesoporous molecular sieve containing phosphorus and iron, 36 wt.% of pseudo-boehmite, 8 wt.% of alumina sol and the balance of kaolin.

### Example 1

The experiment was carried out using the apparatus shown in Fig. 1, wherein the structure of the catalytic cracking reactor can be found as reactor 302 in Fig. 4 of CN 111718230A.

The production process of the biomass alcohol-based fuel comprises the following steps:
In the biomass treatment unit 300, the biomass was subjected to steam explosion pretreatment. The pretreated biomass was subjected to acid hydrolysis. The hydrolysate was conveyed to a fermentation device and fermented at 35 °C to obtain fermentation liquor. The fermentation liquor was subjected to dehydration treatment to obtain the alcohol-based fuel, wherein the alcohol content is 90%, the water content is 3%, and the balance is methanol and C₃₋₅ saturated monohydric alcohol based on the total weight of the alcohol-based fuel.

The raw material oil A was used as a reaction raw material, and the catalytic conversion catalyst a was used as a catalyst. The spent catalyst was regenerated according to the method of the application: the spent catalyst was conveyed from the spent catalyst delivery sloped tube to the first regenerator and contacted with air and alcohol-based fuel (the weight ratio of the spent catalyst to the alcohol-based fuel is 60: 1), which was introduced into the first regenerator from the bottom of the first regenerator through a distribution pipe so that a combustion reaction was generated. The flue gas entered a flue gas energy recovery system after being subjected to a cyclone separation. Part of the regenerated catalyst entered a second regenerator through a riser tube for continuous regeneration. The flue gas entered a flue gas energy recovery system after being subjected to a cyclone separation. The excess energy generated by the regenerator was used for supplying energy to the outside through the heat extractor.

The operating temperature of the first regenerator was 640 °C, the average catalyst residence time was 3 minutes, and the superficial linear velocity of the gas was 0.5 m/s. The second regenerator was operated at 680 °C with an average catalyst residence time of 7 minutes and a superficial linear gas velocity of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 3.

### Comparative Example 1

The experiment was performed with reference to example 1, except that instead of using a biomass treatment unit, diesel was introduced from the first regenerator as fuel oil, which served as a supplemental source of energy. The temperature of the first regenerator was 640 °C, the average catalyst residence time was 3 minutes, and the superficial linear velocity of the gas was 0.5 m/s. The second regenerator has a temperature of 680 °C, an average catalyst residence time of 7 minutes, and a superficial linear velocity of the gas of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 3.

**Table 3 Operating conditions and Results of Example 1 and Comparative Example 1**

| Item | Example 1 | Comparative Example 1 |
|---|---|---|
| Hydrocarbon cracking unit | | |
| Catalyst | a | a |
| Raw material | Raw material oil A | Raw material oil A |
| Reaction temperature, °C | 580 | 580 |
| Catalyst-to-oil ratio | 8.0 | 8.0 |
| Space velocity, h⁻¹ | 10 | 10 |

| Product distribution, wt.% | | |
|---|---|---|
| Dry gas | 5.35 | 5.21 |
| Liquefied gas | 17.66 | 17.33 |
| Propylene | 8.29 | 8.41 |
| Gasoline | 15.68 | 15.71 |
| Diesel oil | 56.93 | 57.76 |
| Catalytic cracking distillate oil | 3.58 | 3.16 |
| Coke | 0.80 | 0.83 |
| Total | 100 | 100 |

| Regeneration unit | | |
|---|---|---|
| First regenerator temperature, °C | 640 | 640 |
| First regenerator residence time, minute | 3 | 3 |
| Second regenerator temperature, | 680 | 680 |
| °C | | |
| Second regenerator residence time, minute | 7 | 7 |
| Supplemental energy combustion medium | Bio-based alcohol-based fuel | Fuel oil |
| Supplemental amount ^{†} | 13.311 g | 7.792 g |

| Regeneration system carbon dioxide emissions index ^{‡} | | |
|---|---|---|
| g CO₂ /MJ | 7.87 | 78.62 |

| | | |
|---|---|---|
| †: Based on 100g of the Raw material processed. ‡: The carbon dioxide emission index refers to the amount of carbon dioxide derived from fossil energy emitted when 1 MJ energy is generated by the coke burning of a regeneration system. The calculation method refers to the "Greenhouse gas emissions accounting methods and reporting guidelines for China's petrochemical enterprises (trial implementation)" (the same below). The carbon dioxide produced by bio-based alcohol-based fuels is derived from carbon dioxide present in the atmosphere and is a neutral carbon emission process. | | |

It can be observed from the data in Table 3 that when the bio-based alcohol-based fuel was used as a source of supplemental energy in example 1, the regeneration system generated the same amount of energy, and the amount of carbon dioxide discharged was significantly reduced compared with that of comparative example 1, which is advantageous for fundamentally reducing the amount of carbon dioxide discharged.

### Example 2

The experiment was carried out using the apparatus shown in Fig. 2, wherein the structure of the catalytic cracking reactor can be found as reactor 302 in Fig. 4 of CN 111718230A.

The production process of the biomass oil includes the following steps:
In processing unit 500, the biomass was ground and crushed, the pretreated biomass was subjected to hydrothermal liquefaction. The liquefaction device has a temperature of 300 °C, a pressure of 12MPa, and a residence time of 10 minutes. The biomass oil fuel was obtained after the liquefaction product was dehydrated.

C₅-C₈ mixed olefin was a reaction raw material (a molar ratio of C₅:C₆:C₇:C₈ olefin is 1: 1: 1: 1). The catalytic conversion catalyst b was a catalyst. The method of the application was used for regenerating the spent catalyst: in a mixing tank on a spent routin, biomass oil was sprayed on a catalyst; they were mixed evenly, wherein the weight ratio of the spent catalyst to the introduced biomass oil was 135:1; the spent catalyst mixed with the biomass oil was introduced into a regenerator to contact with pure oxygen gas diluted by the circulating flue gas, and a combustion reaction occurred. Firstly, a partial regeneration occurred in a coke-burning section, then it ascended to a dense phase regeneration section to complete regeneration, simultaneously a portion of flue gas from a cyclone separation system of a regenerator was returned to the coke-burning section and the dense phase regeneration section, wherein the oxygen content in the coke-burning section and the dense phase regeneration section was controlled not to exceed 28%. The excess energy generated by the regenerator was used for supplying energy to the outside through the heat extractor.

The coke-burning section has an operation temperature of 640 °C, an average catalyst residence time of 90 seconds, and a superficial linear velocity of the gas of 1.0 m/s. The dense phase regeneration section has an operation temperature of 655 °C, an average catalyst residence time of 1.5 minutes, and a superficial linear velocity of the gas of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 4.

### Comparative Example 2

The experiment was carried out with reference to example 2, except that instead of employing a biomass treatment unit, diesel oil was used as a supplemental source of energy, introduced from the coke-burning section. The coke-burning section has a temperature of 640 °C, an average catalyst residence time of 90 seconds, and a superficial linear velocity of the gas of 1.0 m/s. The dense phase regeneration section has a temperature of 655 °C, an average catalyst residence time of 1.5 minutes, and a superficial linear velocity of the gas of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 4.

**Table 4 operating conditions and results of example 2 and comparative example 2**

| Item | Example 2 | Comparative Example 2 |
|---|---|---|
| Hydrocarbon cracking unit | | |
| Catalyst | b | b |
| Raw material | C₅-C₈ Mixed olefins | C₅-C₈ Mixed olefins |
| Reaction temperature, °C | 680 | 680 |
| Reaction time, second | 10 | 10 |
| Catalyst-to-oil ratio | 30 | 30 |

| Product distribution, wt.% | | |
|---|---|---|
| Dry gas | 26.98 | 27.91 |
| Liquefied gas | 57.45 | 56.79 |
| Propylene | 34.09 | 33.91 |
| BTX | 2.34 | 2.78 |
| Light oil | 12.81 | 12.43 |
| Coke | 0.42 | 0.39 |
| Total | 100 | 100 |
| Ethylene + propylene + butylene | 74.21 | 74.21 |

| Regeneration unit | | |
|---|---|---|
| Temperature of the coke-burning | 640 | 640 |
| section,°C | | |
| Average residence time of the coke-burning section, second | 90 | 90 |
| Temperature of the dense phase regeneration section, °C | 655 | 655 |
| The average residence time of the dense phase regeneration section, minute | 1.5 | 1.5 |
| Supplementary heat medium | Biomass oil | Fuel oil |
| Supplemental amount ^{†} | 22.101 g | 11.320 g |

| Regeneration system carbon dioxide emissions index ^{‡} | | |
|---|---|---|
| g CO₂ /MJ | 2.84 | 78.02 |

| | | |
|---|---|---|
| †: Based on 100g of the Raw material processed. ‡: The carbon dioxide emission index refers to the amount of carbon dioxide derived from fossil energy emitted when 1 MJ energy is generated by the coke burning of a regeneration system. The carbon dioxide produced by biomass oil comes from carbon dioxide existing in the atmosphere and is a neutral carbon emission process. | | |

It can be observed from the data in Table 4 that when the light feedstock is used as the cracking feedstock, the amount of carbon deposition is low and far from meeting the energy requirement of the device. When biomass oil is used as the source of the supplementary energy in example 2, the regeneration system generates the same amount of energy, the amount of carbon dioxide discharged is significantly reduced compared with that of comparative example 2, which is beneficial to fundamentally reduce the discharge amount of carbon dioxide.

### Example 3

The experiment was carried out using the apparatus shown in Fig. 3, wherein the structure of the catalytic cracking reactor can be found as reactor 302 in Fig. 4 of CN 111718230A.

Example 3 employed bio-based glycerol as liquid phase fuel. The bio-based crude glycerol comprises 65 wt.% of glycerol, 13 wt.% of methanol, 2 wt.% of methyl oleate, and the balance of water.

Raw material oil A was used as a reaction raw material, and a catalytic conversion catalyst a was used as a catalyst. A coked catalyst and a first product was obtained after the raw material oil A reacted with the catalyst a. The coked catalyst entered a stripping section and contacted with desalted bio-based crude glycerol (the weight ratio of the spent catalyst to the bio-based crude glycerol was 22: 1). A second product was obtained after the reaction. Meanwhile, a portion of the oil gas in the catalyst was stripped. The second product and the stripped oil gas entered a gas collection chamber and entered a fractionating device together with the first product, where they are separated according to the distillation range into products. Unreacted bio-based crude glycerol and heavier products and spent catalyst entered a regenerator through a spent catalyst delivery sloped tube, and contacted with oxygen diluted by circulating flue gas to occur a coke combustion reaction. Firstly, a partial regeneration was carried out in a first regenerator, and conveyed to a second regenerator through a semi-regenerated catalyst pipe to finish regeneration. Meanwhile, a portion of the flue gas separated from a cyclone separation system of the regenerator was returned to the regenerator. The oxygen content in the regenerator was controlled not to exceed 28%. The excess energy generated by the regenerator was used for supplying energy to the outside through the heat extractor.

The first regenerator has an operating temperature of 640 °C, an average catalyst residence time of 2 minutes, and a superficial linear velocity of the gas of 0.5 m/s. The second regenerator has an operating temperature of 670 °C, an average catalyst residence time of 3 minutes, and a superficial linear velocity of the gas of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 5.

### Comparative Example 3

The experiment was carried out with reference to Example 3, except that instead of injecting bio-based glycerol into the stripping section, a same amount of stripping water steam as the bio-based glycerol used in Example 3 was injected. In addition, the heat balance is satisfied by spraying diesel oil as fuel oil into the bottom of the first regenerator to supplement heat. The first regenerator has an operating temperature of 640 °C, an average catalyst residence time of 2 minutes, and a superficial linear velocity of the gas of 0.5 m/s. The second regenerator has an operating temperature of 670 °C, an average catalyst residence time of 3 minutes and a superficial linear velocity of the gas of 0.5 m/s. The regenerated catalyst was circulated back to the reactor and contacted with the raw material oil to carry out a catalytic cracking reaction. The regeneration conditions, reaction conditions, and carbon dioxide emissions are shown in Table 5.

**Table 5 operating conditions and results of Example 3 and Comparative Example 3**

| Item | Example 3 | Comparative Example 3 |
|---|---|---|
| Hydrocarbon cracking unit | | |
| Catalyst | a | a |
| Raw material | Raw material oil A | Raw material oil A |
| Reaction temperature, °C | 600 | 600 |
| Catalyst-to-oil ratio | 8.0 | 8.0 |
| Space velocity, h⁻¹ | 10 | 10 |
| Stripping section injection | Bio-based crude glycerol | / |
| Injection amount | 36.102 g | / |

| Product distribution, wt.% | | |
|---|---|---|
| Dry gas | 12.57 | 8.61 |
| Ethylene | 8.16 | 4.10 |
| Liquefied gas | 21.21 | 20.87 |
| Propylene | 11.54 | 10.30 |
| Gasoline | 14.77 | 15.67 |
| Diesel oil | 47.30 | 50.52 |
| Catalytic cracking distillate oil | 3.03 | 3.17 |
| Coke | 1.13 | 1.16 |
| Total | 100 | 100 |

| Regeneration unit | | |
|---|---|---|
| First/second regenerator temperature, °C | 640/670 | 640/670 |
| First/second regenerator average residence time, minute | 2.0/3.0 | 2.0/3.0 |
| Heat-supplementing bio-based crude glycerol/g | 21.661 | / |
| Heat-supplementing fuel oil consumption/g | / | 7.925 |

| Regeneration system carbon dioxide emissions index^{‡} | | |
|---|---|---|
| g CO₂ /MJ | 10.32 | 78.92 |

| | | |
|---|---|---|
| †: Based on 100g of the Raw material processed. ‡: CO₂ emissions index refers to the amount of fossil energy source-derived CO₂ emitted per 1 MJ of energy generated by the regeneration system coke burning; bio-based liquid phase fuel combustion is a neutral carbon emission process. | | |

It can be observed from the data in table 5 that, in example 3, the bio-based crude glycerol is injected into the stripping section instead of steam, the yield of the low-carbon olefins and the like in the product is increased, and when the regeneration system generates the same energy, the emission amount of carbon dioxide is significantly reduced compared with that of comparative example 3, which is beneficial to fundamentally reducing the emission amount of carbon dioxide.

The preferred embodiments of the present application have been described in detail, however, the present application is not limited to the specific details of the above embodiments, and various simple modifications may be made to the technical solution of the present application within the technical idea of the present application, and these simple modifications are all within the protection scope of the present application.

It should also be noted that the various specific technical features described in the above specific embodiments can be combined in any suitable manner without contradiction. In order to avoid unnecessary repetition, this application will not further describe various possible combinations.

In addition, the various implementation modes of the present application may be arbitrarily combined, and as long as they do not violate the concept of the present application, they should also be regarded as the contents disclosed in the present application.

## Claims

1. A catalyst regeneration method suitable for use in a fluidized catalytic cracking unit comprising a catalytic cracking reactor and a catalyst regenerator, the regeneration method comprising the steps of:
1) providing a bio-based liquid phase fuel;
2) introducing the bio-based liquid phase fuel into a catalyst regenerator or a stripping section of a catalytic cracking reactor;
3) introducing an oxygen-containing gas into the catalyst regenerator, wherein the oxygen-containing gas has an oxygen content of from 14 to 28 volume%, preferably the oxygen-containing gas is selected from air and diluted oxygen, which is diluted by recycled flue gas; and
4) sending the spent catalyst from the catalytic cracking reactor to a catalyst regenerator, where the spent catalyst is contacted with the bio-based liquid phase fuel or the residue thereof and oxygen-containing gas to carry out coke burning regeneration,
preferably, the catalyst regenerator has an operating temperature of 550-750 °C and an average catalyst residence time of 1.0-20.0 minutes.

2. The process according to claim 1, wherein the ratio between the amount of spent catalyst and the amount of bio-based liquid phase fuel introduced in step 2) is 5-400: 1 in mass ratio.

3. The process according to claim 1 or 2, wherein in step 2), the bio-based liquid phase fuel is directly injected into the catalyst regenerator through a distributor or the bio-based liquid phase fuel is injected into a mixing tank provided on a spent catalyst delivery sloped tube for transporting a spent catalyst, and is pre-mixed with the spent catalyst from the catalytic cracking reactor and then is fed into the catalyst regenerator together with the spent catalyst.

4. The process according to claim 1 or 2, wherein in step 2), the bio-based liquid phase fuel is injected into a stripping section of the catalytic cracking reactor, it contacted and reacted with the coked catalyst, and the residual part after the reaction is sent to a catalyst regenerator along with the spent catalyst.

5. The process according to any one of claims 1-4, wherein the catalyst regenerator is a single-stage regenerator and the operating conditions of the regenerator include a temperature of 570-750 °C, an average catalyst residence time of 3-20 minutes, and a superficial linear velocity of the gas of 0.4-1.8 m/s.

6. The process according to any one of claims 1 to 4, wherein the catalyst regenerator is a two-stage regenerator comprising a coke-burning section and a regeneration section in fluid communication, and in step 2) the bio-based liquid phase fuel is introduced into the stripping stage of the catalytic cracking reactor or into the coke-burning and/or regeneration section of the regenerator, in step 3) an oxygen-containing gas is introduced into the bottoms of the coke-burning section and the regeneration section, respectively, and in step 4) the spent catalyst is fed into the coke-burning section,
preferably, the operating conditions of the coke-burning section include an operation temperature of 560 °C-720 °C, an average catalyst residence time of 10-150 seconds, a superficial linear velocity of the gas of 0.8-3.0 m/s, and
the operating conditions of the regeneration section include an operation temperature of 580-750 °C, an average catalyst residence time of 1.0-5.0 minutes, a superficial linear velocity of the gas of 0.3-0.8 m/s;
further preferably, the operating temperature of the regeneration section is 10-150 °C higher than the operating temperature of the coke-burning section.

7. The process according to any one of claims 1 to 4, wherein the catalyst regenerator is a double regenerator comprising a first regenerator and a second regenerator in fluid communication, and in step 2) the bio-based liquid phase fuel is introduced into the stripping section of the catalytic cracking reactor, the first regenerator and/or the second regenerator, in step 3) an oxygen-containing gas is introduced into the bottom of the first regenerator and the bottom of the second regenerator respectively, and in step 4) the spent catalyst is sent to the first regenerator,
preferably, the operating conditions of the first regenerator include an operation temperature of 550-720 °C, an average catalyst residence time of 1.0-5.0 min, and a superficial linear velocity of the gas of 0.4-1.0 m/s; and
the operating conditions of the second regenerator include an operation temperature of 570-750 °C, an average catalyst residence time of 1.0-10.0 minutes, and a superficial linear velocity of the gas of 0.3-0.8 m/s;
further preferably, the operating temperature of the second regenerator is 10-150 °C higher than the operating temperature of the first regenerator.

8. The process according to any one of claims 1 to 7, wherein said step 1) further comprises subjecting biomass to a liquefaction treatment to obtain said bio-based liquid phase fuel, wherein said liquefaction treatment is selected from the group consisting of hydrolytic fermentation, pyrolysis, hydrothermal liquefaction, and alcohol thermal liquefaction, and said bio-based liquid phase fuel is selected from the group consisting of alcohol-based fuels and biomass oil,
preferably, said step 1) comprises one or more of:
performing acid hydrolysis or enzyme hydrolysis on the biomass, and performing microbial fermentation on the obtained hydrolysate at a fermentation temperature of 35-50 °C, wherein the microorganisms are selected from bacteria, fungi, and yeasts, to obtain the water-containing alcohol-based fuel;
carrying out fast pyrolysis or flash pyrolysis on the biomass, wherein the heating rate is 100-200 °C/s, and the residence time is 2-10s, to obtain biomass oil; and
carrying out hydrothermal liquefaction or alcohol thermal liquefaction treatment on the biomass, wherein the treatment temperature is 200-350 °C and the pressure is 4.0-7.0MPa, wherein the solvent for the alcohol thermal liquefaction treatment is selected from methanol and glycol to obtain the biomass oil.

9. The process according to claim 8, wherein step 1) further comprises, prior to said liquefaction treatment, subjecting the biomass to a pretreatment selected from one or more of crushing, drying, torrefaction, compression molding, ball milling, microwave treatment, acid treatment, alkali treatment, steam explosion, carbon dioxide explosion, and microbial degradation.

10. The process according to any one of claims 1 to 9, wherein in step 2), the bio-based liquid phase fuel is introduced into a catalyst regenerator or a stripping section of a catalytic cracking reactor along with other sources of bio-based products, which is by-product crude glycerol from the biodiesel industry, the grease saponification industry and/or the fatty alcohol industry, the crude glycerol comprises 10-90 wt.% glycerol, 1-30 wt.% methanol and 1-30 wt.% fatty acids or fatty acid esters.

11. A catalyst regeneration system suitable for use in a fluidized catalytic cracking unit, comprising a biomass treatment unit and a catalyst regeneration unit, wherein:
the biomass treatment unit is used for liquefying biomass to obtain a bio-based liquid phase fuel, and comprises a biomass liquefaction treatment device, a dewatering device and a storage tank, wherein the biomass liquefaction treatment device is preferably selected from a biomass hydrolysis and fermentation device, a biomass pyrolyzer, a biomass hydrothermal liquefier and a biomass alcohol thermal liquefier, or a combination thereof, and is provided with a biomass inlet and a liquid phase product outlet, the liquid phase product outlet of the biomass liquefaction treatment device is communicated with the inlet of the dewatering device, and the outlet of the dewatering device is communicated with the inlet of the storage tank;
the catalyst regeneration unit is used for regenerating spent catalyst from a catalytic cracking reactor and comprises a catalyst regenerator having a spent catalyst inlet, an oxygen-containing gas inlet, an optional liquid phase fuel inlet, a regenerated flue gas outlet, and a regenerated catalyst outlet, and
the outlet of the storage tank is communicated with the liquid phase fuel inlet of the catalyst regenerator or with the stripping section of the catalytic cracking reactor.

12. The catalyst regeneration system of claim 11, wherein the biomass treatment unit further comprises a biomass pretreatment equipment, which is used for pretreating biomass, wherein the pretreating is selected from one or more of crushing, drying, torrefaction, compression molding, ball milling, microwave treatment, acid treatment, alkali treatment, steam explosion, carbon dioxide explosion, and microbial degradation.

13. The regeneration system of claim 11 or 12, further comprising a spent catalyst delivery sloped tube communicating the catalytic cracking reactor with a spent catalyst inlet of the catalyst regenerator, wherein:
the outlet of the storage tank is communicated with the stripping section of the catalytic cracking reactor, so that the bio-based liquid phase fuel from the storage tank enters the stripping section, and then its reaction residue is conveyed to the spent catalyst inlet of the catalyst regenerator together with the spent catalyst through the spent catalyst delivery sloped tube; or alternatively
a mixing tank is disposed on the spent catalyst delivery sloped tube, and an outlet of the storage tank is communicated with the mixing tank so that the bio-based liquid phase fuel from the storage tank and the spent catalyst are mixed in the mixing tank and then are conveyed to a spent catalyst inlet of the catalyst regenerator through the spent catalyst delivery sloped tube.

14. The catalyst regeneration system according to any one of claims 11-13, wherein the catalyst regenerator comprises a coke-burning section and a dense phase regeneration section, the dense phase regeneration section being located above the coke-burning section and an outlet of the coke-burning section being contained at the internal of the dense phase regeneration section such that the coke-burning section is in fluid communication with the dense phase regeneration section;
the burning section is provided with:
a first oxygen-containing gas inlet disposed at the bottom of the coke-burning section for inputting oxygen-containing gas to the coke-burning section;
an optional first liquid phase fuel inlet and an optional second liquid phase fuel distributor, which is disposed above the first oxygen-containing gas inlet, and is used for direct input of the bio-based liquid phase fuel from the storage tank into the coke-burning section;
the spent catalyst inlet, which is used for conveying spent catalyst from a catalytic cracking reactor to the interior of the coke-burning section; and
an optional first circulating flue gas inlet, which is used for circulating a portion of the flue gas exited from the dense phase regeneration section back into the interior of the coke-burning section;
the dense phase regeneration section is provided with:
a second oxygen-containing gas inlet, which is disposed at the bottom of the dense phase regeneration section, and is used for introducing an oxygen-containing gas into the dense phase regeneration section;
an optional second liquid phase fuel inlet and an optional second liquid phase fuel distributor, which is disposed above the second oxygen-containing gas inlet, and is used for directing the bio-based liquid phase fuel from the storage tank into the dense phase regeneration section;
the regenerated flue gas outlet, which is disposed at the top of the dense phase regeneration section and is used for discharging the regenerated flue gas in the dense phase regeneration section;
the regenerated catalyst outlet, which is used for returning the regenerated catalyst to the catalytic cracking reactor; and
an optional second circulating flue gas inlet, which is used for circulating a portion of the flue gas exited from the dense phase regeneration section back into the interior of the dense phase regeneration section;
optionally, the dense phase regeneration section is further configured with a heat extraction system comprising one or more internal and/or external heat extractors for controlling the temperature of the dense phase regeneration section.

15. The catalyst regeneration system according to any one of claims 11-13, wherein the catalyst regenerator comprises a first regenerator and a second regenerator, the second regenerator is disposed downstream of the first regenerator, the first and second regenerators are connected by a catalyst transfer line, the catalyst material partially regenerated by the first regenerator is transferred to the second regenerator;
the first regenerator is provided with:
a first oxygen-containing gas inlet, which is disposed at the bottom of the first regenerator, and is used for inputting an oxygen-containing gas to the first regenerator;
an optional first liquid phase fuel inlet and an optional first liquid phase fuel distributor disposed above the first oxygen-containing gas inlet for feeding the bio-based liquid phase fuel from the storage tank directly into a first regenerator;
the spent catalyst inlet, which is used for conveying the spent catalyst from the catalytic cracking reactor to the interior of the first regenerator; and
the first regenerated flue gas outlet, which is disposed at the top of the first regenerator and is used for discharging the regenerated flue gas in the first regenerator;
the second regenerator is provided with:
a second oxygen-containing gas inlet, which is disposed at the bottom of the second regenerator and is used for inputting oxygen-containing gas to the second regenerator;
an optional second liquid phase fuel inlet and an optional second liquid phase fuel distributor, which is disposed above the second oxygen-containing gas inlet, and is used for feeding the bio-based liquid phase fuel from the storage tank directly into a second regenerator;
the regenerated catalyst outlet, which is used for returning the regenerated catalyst to the catalytic cracking reactor; and
a second regenerated flue gas outlet, which is disposed at the top of the second regenerator, and is used for discharging the regenerated flue gas in the second regenerator,
optionally, the first and second regenerators are further configured with a heat extraction system comprising one or more internal and/or external heat extractors for controlling the temperature of the first and second regenerators.
